# EUROPÄISCHE PATENTANMELDUNG

(11) **EP 0 918 216 A2**
(43) Veröffentlichungstag der Anmeldung: **26.05.1999**
(21) Anmeldenummer: 98118852.7
(22) Anmeldetag: 06.10.1998
(51) Int. Cl.: G01N 33/00

(54) **Verfahren und Einrichtung zur Aufbereitung eines Messgases**

(30) Priorität: 22.11.1997 DE 19751892
(71) Anmelder: Hartmann & Braun GmbH & Co. KG, 65760 Eschborn (DE)
(72) Erfinder: Fabinski, Walter, Dipl.-Ing., 65830 Kriftel (DE); Hielscher, Bernd Dr., 61118 Bad Vilbel (DE)

(57) **Zusammenfassung**

Die Erfindung betrifft ein Verfahren sowie eine Einrichtung zur Aufbereitung eines Probengases, bei welchem das Messgas zur Befreiung von messgasbegleitenden Gas- und/oder Wasserdampfanteilen in einem Kühler gekühlt wird. Um bei einem Verfahren dieser Art den beschriebenen Bleikammereffekt zu unterdrücken und gleichzeitig eine verlustminimierte Kondensatabscheidung (LowLoss) zu erhalten ist erfindungsgemäß vorgeschlagen, daß das Messgas nach Durchlaufen der Kühlstrecke und Abscheidung des Kondensates wieder aufgeheizt wird.

## Beschreibung

Die Erfindung betrifft ein Verfahren sowie eine Einrichtung zur Aufbereitung eines Meßgases, bei welchem das Meßgas zur Befreiung von begleitenden Gas- und/oder Wasserdampfanteilen in einem Kühler gekühlt wird, gemäß Oberbegriff der Patentansprüche 1 und 5.

In der nicht vorveröffentlichten Schrift DE 196 23 894 wird ein Kühler zur Entfernung von Kondensat aus dem Meßgas beschrieben. Das Meßgas wird vor dem Kühler beheizt und verläßt den Kühler mit ca. 2°C Taupunkt und derselben Eigentemperatur. Bei diesem Kühler ist es das Ziel, die Verluste durch Lösung im Kondensat gering zu halten. Der im Kondensat gelöste Anteil entspricht hier der Lösungstemperatur der Beheizung vor dem Kühler. Bei diesem Kühler hat das den Kühler verlassende Gas eine Eigentemperatur von ca. 2°C, der Temperatur des Kühlers.

Eine tiefe Kühlertemperatur beschleunigt aber den sog. Bleikammereffekt. Dieser tritt auf, wenn sich NO und SO2 bei Feuchte im Meßgas befinden. Unter diesen Bedingungen entsteht Schwefelsäure, die in den Meßgasleitungen bis hin zur Meßzelle Schädigungen hervorruft und das Meßergebnis verfälschen kann.

Der Erfindung liegt daher die Aufgabe zugrunde, den beschriebenen Bleikammereffekt zu unterdrücken und gleichzeitig eine verlustminimierte Kondensatabscheidung (LowLoss) zu erhalten.

Die gestellte Aufgabe wird hinsichtlich eines Verfahrens der gattungsgemäßen Art erfindungsgemäß durch die kennzeichnenden Merkmale des Patentanspruches 1 gelöst. weitere vorteilhafte Ausgestaltungen des erfindungsgemäßen Verfahrens sind in den abhängigen Ansprüchen 2 bis 4 dargestellt.

Hinsichtlich einer Einrichtung der gattungsgemäßen Art wird die gestellte Aufgabe erfindungsgemäß durch die kennzeichnenden Merkmale des Patentanspruches 5 gelöst. Weitere vorteilhafte Ausgestaltungen der Einrichtung sind in den übrigen Patentansprüchen angegenben.

Die Erfindung hat zum Ziel, den Bleikammereffekt zu unterdrücken oder auszuschalten bei gleichzeitigem Erhalt der Eigenschaft der verlustminimierten Kondensatabscheidung (LowLoss) Dies geschieht in erfindungsgemäßer Weise dadurch, daß das Meßgas unmittelbar nach der Kühlung wieder erwärmt wird. Zugrundegelegt wird dabei die Tatsache, daß der Bleikammereffekt bei ca. 60°C 10x so langsam läuft wie bei 2°C (LowCorrosion)

Dies wird gemäß der Erfindung erreicht, indem bei dem das Probengas unmittelbar vor dem Kühler in einem ersten Schritt beheizt und dann im Gegenstromverfahren in einer Kühlstrecke weitgehend verlustfrei vom Kondensat H2O befreit wird. Nach der Abkühlung wird das Gas dann in einem 2. Schritt wieder entgegen dem Meßgasstrom zunächst von dem gekühlten Meßgas und weiter der Heizeinrichtung so beheizt, daß es den Kühler mit einer Taupunkt von ca. 2°C und einer Eigentemperatur von vorwiegend 40-80°C und einer bei ca. 80°C gelösten Meßkomponente verläßt.

Bei der vorliegenden Erfindung wird das Meßgas dazu im Ausgang wieder beheizt, um den Bleikammereffekt zu unterdrücken. Eine besondere Variante benutzt dazu eine Anordnung, in der das Meßgas bei Eintritt in den Kühler zur Unterdrückung von Löslichkeit der Meßkomponente beheizt wird. Erfindungsgemäß kann dieselbe Heizung auch zum Beheizen des austretenden Meßgases eingesetzt werden. Damit kann der Kühler sowohl Meßgasverluste im Kondensat minimieren als auch den Bleikammereffekt unterdrücken.

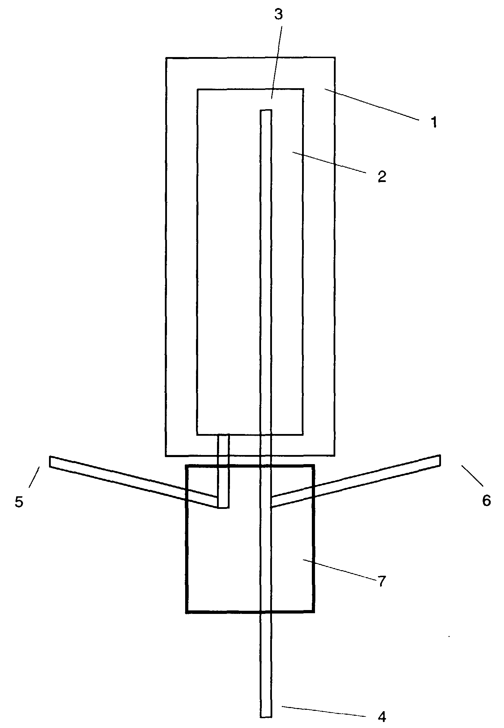

Die Erfindung ist in der Abbildung dargestellt und nachfolgend näher beschrieben.

Die Abbildung zeigt einen erfindungsgemäßen Kühler welcher hier im Gegenstrom betrieben wird. Auf diese Weise erfolgt der Meßgaseintritt sowie der Meßgasaustritt an einer gemeinsamen Seite des Kühlers. Der Kühler selbst besteht in einfachster Ausführung aus einem Kühlgefäß 1 innerhalb dessen ein Proben- oder Meßgasgefäß 2 angeordnet ist. Das Meßgasgefäß 2 ist gaseingansseitig mit einem in das Meßgasgefäß 2 weit hineinreichenden Rohr 3 versehen. Durch dieses Rohr 3 wird das eintretende Meßgas quasi bis zum anderen Ende des Meßgasgefäßes hineingebracht, strömt von dort aus diesem Rohr 3 in das Meßgasgefäß 2 hinein, und strömt - im Gegenstrom - nun wieder zurück zur Austrittstelle, wo es über das Austrittsrohr 5 aus dem Kühler austritt. Vor Eintritt des Probengases in den Kühler wird dasselbe in der Vorwärmstufe vorgewärmt. Nach Austritt des gekühlten Meßgases wird dasselbe wieder aufgeheizt, um den beschriebenen Bleikammereffekt zu unterbinden. Meßgaseintritt 6 sowie Meßgasaustritt 5 sind baulich so nahe beieinanderliegend angeordnet, daß Vorwärmstufe und Wiedererwärmungsstufe baulich zusammengefasst und innerhalb eines gemeinsamen Heizblockes 7 angeordnet sind. Dadurch wird die gesammte Einrichtung zudem sehr kompakt und macht nur einen Heizblock notwendig.

## Patentansprüche

1. Verfahren zur Aufbereitung eines Meßgases, bei welchem das Meßgas zur Befreiung von begleitenden Gas- und/oder Wasserdampfanteilen vor Einritt in einen Kühler vorgewärmt wird, und sodann in diesem Kühler bis auf einen Taupunkt gekühlt und das Kondensat abgeschieden wird,
**dadurch gekennzeichnet,**
daß das Messgas nach Durchlaufen der Kühlstrecke und Abscheidung des Kondensates wieder aufgeheizt wird.

2. Verfahren zur Aufbereitung eines Meßgases, nach Anspruch 1,
**dadurch gekennzeichnet,**
daß der Durchlauf des Meßgases durch die Kühlstrecke im Gegenstrom erfolgt,
und daß sowohl der Eintritt des Meßgases als auch der Austritt des gekühlten Meßgases räumlich nahe beieinanderliegend erfolgt.

3. Verfahren zur Aufbereitung eines Meßgases, nach Anspruch 2;
**dadurch gekennzeichnet,**
daß sowohl die Vorwärmung als auch die Wiedererwärmung innerhalb eines gemeinsamen Heizelementes vorgenommen wird.

4. Verfahren zur Aufbereitung eines Meßgases, nach einem oder mehreren der vorhergehenden Ansprüche,
**dadurch gekennzeichnet,**
daß das Meßgas auf eine Temperatur bzw Taupunkte zwischen 2°C und 8°C gekühlt wird, und anschließend wieder auf 40°C bis 80°C wiedererwärmt wird.

5. Einrichtung zur Aufbereitung eines Meßgases, bei welchem das Meßgas zur Befreiung von begleitenden Gas- und/oder Wasserdampfanteilen in einer Vorwärmstufe vorgewärmt und nachfolgend in einem Kühler bis auf einen Taupunkt gekühlt und das Kondensat abgeschieden wird
**dadurch gekennzeichnet,**
daß am Meßgasaustritt (5) des Kühlers ein Heizung zur Wiederaufheizung des Meßgases angeordnet ist.

6. Einrichtung zur Aufbereitung eines Meßgases, nach Anspruch 5,
**dadurch gekennzeichnet,**
daß die Gasführung innerhalb des Kühlers gegenstromig ist, und daß der Meßgaseintritt (6) sowie auch der Meßgasaustritt (5) räumlich nahe beieinanderliegend angeordnet sind.

7. Einrichtung zur Aufbereitung eines Meßgases, nach Anspruch 6,
**dadurch gekennzeichnet,**
daß die Vorwärmstufe und die Heizung zur Wiederaufheizung baulich zu einem gemeinsamen Heizblock (7) zusammengefasst sind.
